# EUROPÄISCHE PATENTSCHRIFT

(11) **EP 3 409 678 B1**
(45) Veröffentlichungstag und Bekanntmachung des Hinweises auf die Patenterteilung: **21.04.2021**
(21) Anmeldenummer: 17173959.2
(22) Anmeldetag: 01.06.2017
(51) Int. Cl.: C07F 7/30, C07F 19/00

(54) **NEUE HALOGENGERMANIDE UND VERFAHREN ZU DEREN HERSTELLUNG**
NEW HALOGEN GERMANIDES AND METHOD FOR THEIR PREPARATION
NOUVEAU GERMANIDE HALOGÈNE ET SON PROCÉDÉ DE FABRICATION

(43) Veröffentlichungstag der Anmeldung: 05.12.2018
(73) Patentinhaber: Evonik Operations GmbH, 45128 Essen (DE)
(72) Erfinder: TEICHMANN, Julian, 60327 Frankfurt am Main (DE); WAGNER, Matthias, 61194 Niddatal (DE); LERNER, Hans-Wolfram, 61440 Oberursel (DE)
(74) Vertreter: Evonik Patent Association

(56) Entgegenhaltungen:
- WO-A2-2004/036631
- US-A- 3 657 368
- K FUTTERER ET AL: "The low-temperature phase sequence gamma - delta - epsilon in halide perovskite tetramethylammonium trichlorogermanate(II) studied by X-ray diffraction", JOURNAL OF PHYSICS: CONDENSED MATTER., Bd. 7, Nr. 26, 26. Juni 1995 (1995-06-26), Seiten 4983-4998, XP055427800, GB ISSN: 0953-8984, DOI: 10.1088/0953-8984/7/26/005
- B. WINKLER ET AL: "Dynamics of N(CH3)4GeCl3", PHYSICA B: CONDENSED MATTER, Bd. 234-236, 1. Juni 1997 (1997-06-01), Seiten 70-71, XP055427809, AMSTERDAM, NL ISSN: 0921-4526, DOI: 10.1016/S0921-4526(96)00883-6
- POSKOZIM ET AL: "Methylammonium trichlorogermanate(II) salts", METHYLAMMONIUM TRICHLOROGERMANATE(II) SALTS, Bd. 32, Nr. 4, 1. April 1970 (1970-04-01), Seiten 1391-1393, XP002775894,
- GEORGE W. PARSHALL: "Catalysis in molten salt media", JOURNAL OF THE AMERICAN CHEMICAL SOCIETY, Bd. 94, Nr. 25, 1. Dezember 1972 (1972-12-01), Seiten 8716-8719, XP55368902, US ISSN: 0002-7863, DOI: 10.1021/ja00780a013
- Benjamin Peerless ET AL: "Homoleptic low-valent polyazides of group 14 elements", Chemical communications (Cambridge, England), 1. Mai 2015 (2015-05-01), Seiten 7435-7438, XP55427835, England DOI: 10.1039/C5CC00259A Gefunden im Internet: URL:http://www.rsc.org/suppdata/c5/cc/c5cc 00259a/c5cc00259a1.pdf
- CUMMINGS AT AL: "A reference electrode for use in supercritical difluoromethane", ELECTROCHIMICA ACTA, Bd. 187, 1. Dezember 2015 (2015-12-01), Seiten 323-328, XP002775895,
- MULLER L ET AL: "Trichlorosilylation of chlorogermanes and chlorostannanes with HSiCl3/Net3 followed by base-catalysed formation of (Me3Ge)2Si(SiCl3)2 and related branched stannylsilanes", JOURNAL OF ORGANOMETALLIC CHEMISTRY, ELSEVIER-SEQUOIA S.A. LAUSANNE, CH, Bd. 579, Nr. 1-2, 5. Mai 1999 (1999-05-05) , Seiten 156-163, XP004170561, ISSN: 0022-328X, DOI: 10.1016/S0022-328X(98)01218-2

## Beschreibung

Die vorliegende Erfindung betrifft neue Halogengermanide und ein Verfahren zur deren Herstellung.

Halogensilane, Polyhalogensilane, Halogengermane, Polyhalogengermane, Silan, Polysilane, German, Polygermane sowie entsprechende Mischverbindungen sind lange bekannt, vgl. neben den gängigen Lehrbüchern der anorganischen Chemie auch WO 2004/036631 A2 oder C.J. Ritter et al., J. Am. Chem. Soc., 2005, 127, 9855-9864.

L. Müller et al. beschreiben in J. Organomet. Chem., 1999, 579, 156-163, u.a. die Herstellung von Trichlorsilylmethylgermanen.

Aus der Angew. Chem., 1993, 105, 587-588 (G. Sih et al.) sowie aus Tetrahedron Lett., 1970, 51, 4443-4447 (F. Feher et al.) sind Methylgermylsilane sowie Phenylgermylsilane bekannt.

Ferner sind Phenyl- und Wasserstoff-enthaltende Verbindungen bekannt, in denen Sn-Sisowie Sn-Ge-Bindungen vorliegen (J.B. Tice et al., J. Inorganic Chemistry, 2009, 48(13), 6314-6320). Dabei wird angeregt, diese Verbindungen als IR Halbleiter zu verwenden.

Singh et al. offenbaren in der Patentschrift US 7,540,920 B2 Si-Ge Verbindungen der Formel X₁X₂X₃-Si-Ge- X₄X₅X₆ mit Wasserstoff- oder Halogen-Resten X₁₋₆.

Über Chlorsilylarylgermane ist bis heute praktisch nichts bekannt. So ist man seitens der Grundlagenforschung bestrebt, neue Verbindungen aufzufinden und neue Herstellungswege zu suchen, insbesondere auch mit Hinblick auf mögliche industrielle sowie ggf. verbesserbare Anwendungen.

Der vorliegenden Erfindung lag die Aufgabe zugrunde, neue Germanium- bzw. neue Silicium-Germanium Verbindungen sowie ein Verfahren zu deren Herstellung bereit zu stellen.

Neue Synthesemöglichkeiten zur Herstellung von Silicium-Germanium Verbindungen, insbesondere
R₃Ge-SiCl₃, Cl₃Si-GeR₂-GeR₂-SiCl₃, Cl₃Ge-SiCl₃, [Ph₄P][Ge(SiCl₃)₃], [Ph₄P][Ge(SiCl₃)₃*GaCl₃], [Ph₄P][Ge(SiCl₃)₃*BBr₃], die Umsetzung von chlorierten oder perchlorierten, organischen oder nicht organischen Germaniumverbindungen des Typs RₙGeCl₄₋ₙ (n = 0,2,3) mit Hexachlordisilan unter Zugabe von katalytischen Mengen eines Ammoniumchloridsalzes [R₄N]Cl mit den Resten R = Me, Et, *i*Pr, *n*Bu wurden in den Patentanmeldungen mit dem Titel "*Neue Chlorsilylarylgermane*, *Verfahren zu deren Herstellung und deren Verwendung"* bzw. *"Triphenylgermylsilan*, *Verfahren zur Herstellung und seine Verwendung"* vorgestellt. Durch die Umsetzung werden verschiedene Silicium-Germanium Verbindungen erhalten.

Des Weiteren wurde gefunden, dass Edukte des Typs R₃GeCl sich durch die Reaktion mit Si₂Cl₆ in Anwesenheit von Ammoniumchlorid umsetzen, wobei an der Ge-Cl Position eine Ge-Si Bindungsknüpfung stattfindet. Bei der Umsetzung von R₂GeCl₂ mit Si₂Cl₆/Ammoniumchlorid erfolgt die Ge-Si Bindungsbildung nur an einer Ge-Cl Position. Zusätzlich tritt eine Ge-Ge Bindungsknüpfung an der zweiten Ge-Cl Position auf. Die so gefundenen bzw. hergestellten Germanium-Silicium-Verbindungen lassen sich mit LiAlH₄ in Siliciumhydridverbindungen überführen. Solche Verbindungen werden im Rahmen der Erfindung am Beispiel Ph₃Ge-SiCl₃ mit der Synthese von Ph₃Ge-SiH₃ hergestellt. Im Fall der Reaktionen von Verbindungen des Typs RₙGeCl₄₋ₙ (n = 0) finden je nach Stöchiometrie unterschiedliche Reaktionen statt.
Es wurden
[R₄N][GeCl₃]- Salze (GeCl₄:Si₂Cl₆:[R₄N]Cl, 1:1:1),
Cl₃Si-GeCl₃ (GeCl₄:Si₂Cl₆:[R₄N]Cl, 1:1:0.1), oder
[Ph₄P][Ge(SiCl₃)₃] (GeCl₄:Si₂Cl₆:[Ph₄P]Cl, 1:4:1)
erzeugt. [Ph₄P][Ge(SiCl₃)₃] lässt sich mit Lewis-Säuren, abgekürzt "LS", zu den entsprechenden Addukten [Ph₄P][Ge(SiCl₃)₃*LS] umsetzen.

Im Rahmen der Erfindung wird unter dem Maß "equivalent", kurz "eq.", die Menge des Katalysators in mol bezogen auf die Menge Hexachlordisilan in mol verstanden. Beispielsweise bedeutet 0,1 eq. Katalysator 0,1 mol Katalysator pro mol Hexachlordisilan oder 10 mol-% Katalysator bezogen auf Hexachlordisilan.

Im Rahmen der Erfindung wird "Hexachlordisilan" auch mit "HCDS" abgekürzt.

Chlorsilylarylgermane werden hergestellt, indem man
(a) ein Arylchlorgerman mit Hexachlordisilan in einem Lösemittel löst und
(b) in Gegenwart eines Katalysator bei einer Temperatur von 5 bis 40 °C umsetzt, wobei die Aryl-Gruppen des Arylchlorgermans unabhängig voneinander für Phenyl
stehen.

Die Chlorsilylarylgermane können ausgewählt sein aus der Reihe Trichlorsilyltriphenylgerman und/oder 1,2-Bis(trichlorsilyl)-1,1,2,2-tertraphenyldigerman.

Der Schritt (b) des besagten Herstellverfahrens kann bei Raumtemperatur durchgeführt werden.

Im Schritt (a) des Verfahrens kann ein Triarylchlorgerman in einem molaren Verhältnis zu Hexachlordisilan von 1 zu 1 und/oder ein Diaryldichlorgerman in einem molaren Verhältnis zu Hexachlordisilan von 1 zu 2 eingesetzt werden.

Des Weiteren kann ein Arylchlorgerman aus der Reihe Triphenylchlorgerman und/oder Diphenyldichlorgerman eingesetzt werden.

Das im Schritt (a) eingesetzte Lösemittel kann inert gewählt sein, um unerwünschte Umsetzungen mit HCDS zu vermeiden. Weiterhin kann Dichlormethan eingesetzt werden, da dieses in den gewählten Temperaturbereichen nicht mit HCDS reagiert.

Bei der Durchführung des erfindungsgemäßen Verfahrens können als Katalysator Phosphoniumchloride [R₄P]Cl oder Ammoniumchloridsalze [R₄N]Cl eingesetzt werden, wobei die Reste R ausgewählt sind aus Me, Et, *i*Pr, *n*Bu, Ph.

Dabei läuft die Umsetzung umso langsamer ab, je weniger Katalysator eingesetzt wird. Andererseits sind zu große Mengen Katalysator nicht wünschenswert, da dieser am Ende der Umsetzungsreaktion abgetrennt werden muss. Das Verfahren kann wirtschaftlich im Hinblick auf den Abtrennungsaufwand durchgeführt werden, wenn man den Katalysator in einer Menge von 0,001 bis 1 mol pro mol Hexachlordisilan einsetzt. Das Verfahren wird besonders günstig durchgeführt, wenn man von 0,01 bis 0,1 mol pro mol Hexachlordisilan einsetzt.

Ein weiterer Aspekt, das Verfahren wirtschaftlich durchzuführen, ist die Auswahl der Menge des Lösemittels. In dem Verfahren kann man mindestens 5 mol Lösemittel pro mol Hexachlordisilan, bevorzugt von 10 mol bis 100 mol Lösemittel pro mol Hexachlordisilan einsetzen.

In dem Verfahren kann die Umsetzung unter Durchmischung, bevorzugt unter Rühren, und über eine Dauer von 1 bis 24 Stunden, vorzugsweise während 12 h, und weiterhin vorzugsweise unter Schutzgas, bevorzugt unter Stickstoff oder Argon durchgeführt werden. Anschließend kann man das Lösemittel entfernen. Dies kann vorzugsweise in Sauerstoff freier trockener Umgebung vorgenommen werden, besonders bevorzugt in einer isolierten Umgebung, beispielsweise in einer Glovebox, und weiterhin bevorzugt unter Normaldruck oder vermindertem Druck, bevorzugt im Bereich von 1 bis 500 hPa, wobei man Chlorsilylarylgermane als kristallines Produkt gewinnt.

Auch das interessante Molekül Trichlorsilyltrichlorgerman kann hergestellt werden, welches bereits bei Singh et al. (US 7,540,920 B2) ohne eine Methode zur Herstellung offenbart ist. Hierzu wird anstelle des Arylchlorgerman GeCl₄ eingesetzt. Die Umsetzungsreaktion wird dann im Weiteren mit Hexachlordisilan in einem Lösemittel und in Anwesenheit eines Katalysators durchgeführt.

Bei der Herstellung von Trichlorsilyltrichlorgerman löst man GeCl₄ mit Hexachlordisilan in einem Lösemittel und führt die Umsetzung in Gegenwart eines Katalysators bei einer Temperatur von 5 bis 40 °C durch.

Es kann vorteilhaft sein, den Katalysator in Mengen von 0,001 bis 1 eq., vorzugsweise von 0,01 bis 0,1 eq. einzusetzen. Eine beispielhafte Durchführung dieses Verfahrens schildert **Beispiel 3.**

Wird dieses Verfahren weiterhin dadurch modifiziert, indem man das Ammoniumchloridsalz [R₄N]Cl oder Phosphoniumchloridsalze [R₄P]Cl anstelle in katalytischer in stöchiometrischer Menge einsetzt, vorzugsweise von 0,5 bis 1,5 eq., besonders bevorzugt 1 eq., dann werden überraschend Halogengermanide erhalten, nämlich beim Einsatz von [R₄N]Cl Trichlorogermanid.

Gegenstand der Erfindung ist ein Verfahren zur Herstellung von Trichlorogermaniden der allgemeinen Formel I

(I) [R₄N]/[R₄P]Cl[GeCl₃],

mit R = Me, Et, *i*Pr, *n*Bu,
indem man GeCl₄ mit Hexachlordisilan in einem Lösemittel löst und in Gegenwart von [R₄N]Cl oder [R₄P]Cl in stöchiometrischer Menge bei einer Temperatur von 5 bis 40 °C umsetzt.

Unter dem Ausdruck "[R₄N]/[R₄P]Cl" in der Formel I ist im Rahmen der Erfindung zu verstehen, dass Phosphonium- bzw. Ammoniumsalze erhalten werden.

Eine bevorzugte Durchführung mit R = nBu schildert **Beispiel 4.**

Die gemäß **Beispiel 4** durchgeführte Synthese stellt eine neue Möglichkeit zur Gewinnung von [R₄N][GeCl₃]-Salzen bzw. [R₄P]Cl[GeCl₃]-Salzen dar, indem das Kation [R₄N]⁺ bzw. [R₄P]⁺ variiert und das gewünschte Chloridsalz zur heterolytischen Spaltung des Si₂Cl₆ verwendet wird. Dies ist besonders interessant, da gefunden worden ist, dass das Kation darüber entscheidet, wie gut eine Verbindung kristallisiert.

So kann man erfindungsgemäß eine große Vielzahl an [R₄N][GeCl₃]-Salzen bzw. [R₄P]Cl[GeCl₃]-Salzen herstellen.

Setzt man in einer Modfikation des Verfahrens die Ausgangstoffe Si₂Cl₆ und GeCl₄ in einem Verhältnis 4:1 unter Zugabe stöchimetrischer Mengen von [R₄N]Cl oder [R₄P]Cl ein, wird überraschend Tris-Trichlorsilylgermanid erhalten.

Beispielsweise kann man [Ph₄P]Cl einsetzen (**Beispiel 5**).

Gegenstand der Erfindung ist gleichfalls ein Verfahren zur Herstellung von Tris-Trichlorsilylgermanid der Formel II,

(II) [R₄P]/[R₄N][Ge(SiCl₃)₃],

indem man Hexachlordisilan mit GeCl₄ in einem Lösemittel löst und in Gegenwart von [R₄N]Cl oder [R₄P]Cl in stöchiometrischer Menge bei einer Temperatur von 5 bis 40 °C umsetzt.
Ein bevorzugtes Verhältnis von Hexachlordisilan und GeCl₄ ist 4:1.

Als stöchiometrische Menge wird vorzugsweise von 0,5 bis 1,5 eq., besonders bevorzugt 1 eq. [R₄N]Cl oder [R₄P]Cl eingesetzt. Eine beispielhafte Synthese des erfindungsgemäßen Germanids (II) ist in **Beispiel 5** näher erläutert.

Wird das erfindungsgemäß erhaltene Tris-Trichlorsilylgermanid mit GaCl₃ umgesetzt, erhält man Tris(trichlorsilyl)germanid an GaCl₃ Addukt:

[Ph₄P][Ge(SiCl₃)₃*GaCl₃].

Gegenstand der Erfindung ist daher gleichfalls das Verfahren zur Herstellung des Adduktes der Formel III,

(III) [Ph₄P][Ge(SiCl₃)₃*GaCl₃],

indem man das erfindungsgemäß erhaltene Tris-Trichlorsilylgermanid mit GaCl₃ vermischt und das erhaltene Gemisch in Dichlormethan unter Rühren löst, wobei anschließend bei einer Temperatur von 5 bis 40 °C während 1 bis 24 Stunden das Addukt III erhalten wird.

Eine nähere Erläuterung der Synthese ist im **Beispiel 6** angegeben.

Anstelle der erfindungsgemäßen Umsetzung des Tris-Trichlorsilylgermanids mit GaCl₃ kann Tris-Trichlorsilylgermanid mit BBr₃ umgesetzt werden. Hierbei wird Tris(trichlorsilyl)germanid an BBr₃ Addukt gefunden.

Gegenstand der Erfindung ist somit gleichfalls das Verfahren zur Herstellung des Adduktes der Formel IV,

(IV) [Ph₄P][Ge(SiCl₃)₃*BBr₃],

indem man das erfindungsgemäß erhaltene Tris-Trichlorsilylgermanid mit BBr₃ vermischt und das erhaltene Gemisch in Dichlormethan unter Rühren löst, wobei anschließend bei einer Temperatur von 5 bis 40 °C während 1 bis 120 Stunden das Addukt IV erhalten wird.

Eine nähere Erläuterung dieser Synthese ist im **Beispiel 7** angegeben.

Die erfindungsgemäß hergestellten Ge- bzw. Si-Ge Verbindungen können als Vorstufe für Materialien dienen, die für die gezielte Abscheidung von dünnen Si-Ge Schichten eingesetzt werden.

Die nachfolgenden Beispiele erläutern die vorliegende Erfindung zusätzlich, ohne den Gegenstand zu beschränken. Der Begriff "Raumtemperatur" wird in den Beispielen mit "RT" abgekürzt.

### Analytische Methoden der Kristallstrukturbestimmung

Die Daten für alle Strukturen wurden bei 173 K mit einem STOE IPDS II Zweikreisdiffraktometer mit einer Genix Mikrofokusröhre mit Spiegeloptik unter Verwendung von Mo*K*_{α} Strahlung (*λ*= 0,71073 Å) gesammelt und mit dem frame scaling procedure des Programms *X-AREA* (Stoe & Cie, 2002) skaliert. Die Strukturen wurden mit direkten Methoden mit Hilfe des Programms *SHELXS* (Sheldrick, 2008) gelöst und gegen *F*² mit der full-matrix least-squares Technik verfeinert. Zellparameter wurden durch Verfeinerung gegen θ Werte der Reflexe mit *I*>6σ(*I*) bestimmt.

### Einsatzstoffe:

Hexachlordisilan, kurz "HCDS", GeCl₄ von Evonik Industries AG, Triphenylchlorgerman, Diphenyldichlorgerman. (Nur Beispiele, welche unter die Ansprüche fallen, sind erfindungsgemässe Ausführungsbeispiele).

### Beispiel 1: Herstellung von Trichlorsilyl-triphenylgerman (1).

Die Synthese erfolgte gemäß **Gleichung 1** aus Ph₃GeCl und Si₂Cl₆ unter Zugabe einer katalytischen Menge von 0,1 eq [*n*Bu₄N]Cl. 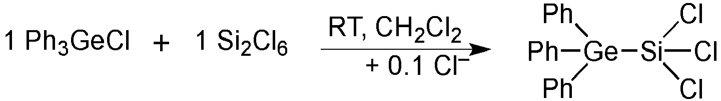 **Gleichung 1**: Umsetzung von Ph₃GeCl und Si₂Cl₆ unter Zugabe der katalytischen Menge von 0,1 eq [*n*Bu₄N]Cl.

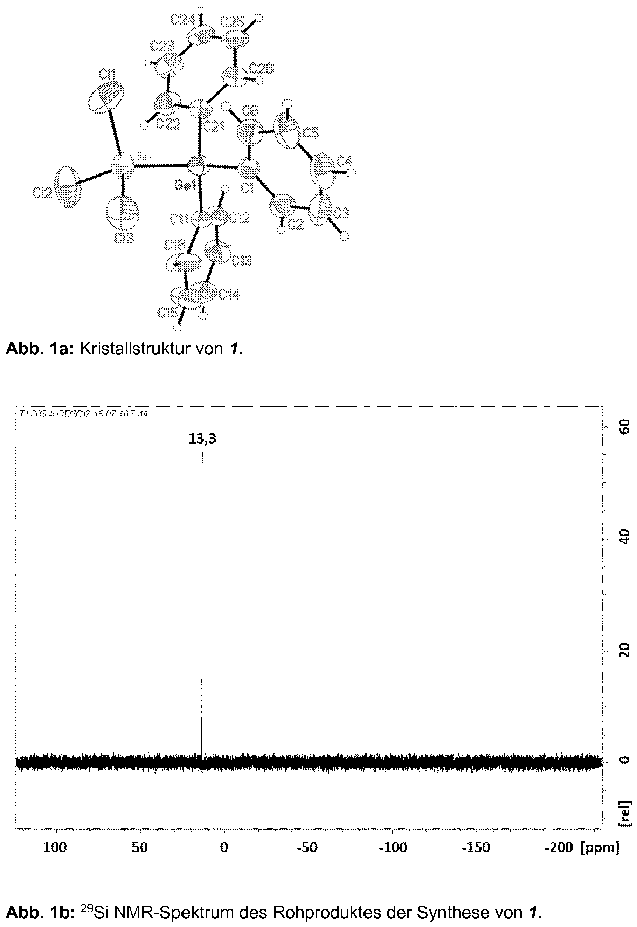

Zu einer klaren, farblosen Lösung aus 500 mg entsprechend 1,47 mmol Ph₃GeCl und 40 mg oder 0,14 mmol [*n*Bu₄N]Cl in 5 ml oder 78,3 mmol CH₂Cl₂ wurden unter Rühren bei Raumtemperatur 400 mg entsprechend 1,49 mmol Si₂Cl₆ gegeben. Es wurde eine klare, farblose Reaktionslösung erhalten, die bei Raumtemperatur während 12 h gerührt wurde. Aus der Reaktionslösung ließ sich nach langsamem Entfernen des Lösemittels ein Rohprodukt in Form eines farblosen, kristallinen Feststoffes ***1*** isolieren. Die Ausbeute betrug 59 %. Das Rohprodukt enthielt noch zu etwa 30 % das Edukt Ph₃GeCl. Durch Röntgendiffraktometrie konnte die Kristallstruktur von ***1*** ermittelt werden (Abb. 1a). Das nicht gekennzeichneten Atome stehen in der Abbildung für Wasserstoff.

Das ²⁹Si NMR Spektrum von ***1*** sind in Abb. 1b dargestellt.
Alle Ergebnisse einer ¹H, ¹³C und ²⁹Si NMR-spektroskopischen Untersuchung:
**²⁹Si NMR (99,4 MHz, CD₂Cl₂, 298 K):**
   δ = 13,3.
**¹H NMR (500,2 MHz, CD₂Cl₂, 298 K):**
   δ = 7,58 (m); 7,75 (dd ³J(H,H) = 8,0 Hz, ²J(H,H) = 1,4 Hz).
**¹³C NMR (125,0 MHz, CD₂Cl₂, 298 K):**
   δ = 128,9; 130,1; 132,2; 135,3.

### Beispiel 2: Herstellung von 1,2-Bis(trichlorsilyl)-1,1,2,2-tetraphenyldigerman (2).

Die Synthese erfolgte gemäß **Gleichung 2** aus Ph₂GeCl₂ und Si₂Cl₆ unter Zugabe von katalytischen Mengen (0.1 eq) [*n*Bu₄N]Cl. 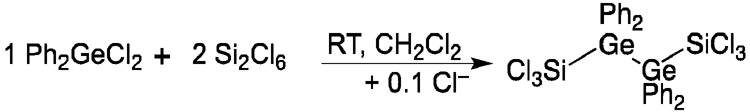 **Gleichung 2:** Umsetzung von Ph₂GeCl₂ und Si₂Cl₆ unter Zugabe von katalytischen Mengen (0.1 eq) [*n*Bu₄N]Cl.

Zu einer klaren, farblosen Lösung enthaltend 500 mg oder 1,68 mmol Ph₂GeCl₂ und 90 mg oder 0,32 mmol [*n*Bu₄N]Cl in 5 ml oder 78,3 mmol CH₂Cl₂ wurde bei Raumtemperatur Si₂Cl₆ gegeben. Die erhaltene Reaktionslösung wurde anschließend während 12 h gerührt.

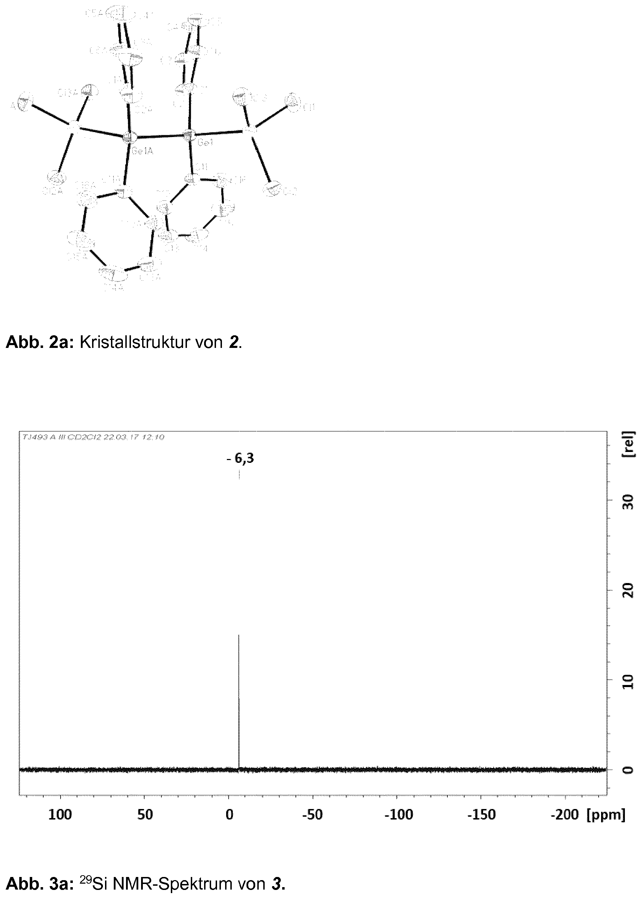

Aus der Reaktionslösung ließ sich nach langsamem Entfernen des Lösemittels 2 als Rohprodukt in einer Ausbeute von 77 % erhalten, aus dem sich durch Extrahieren mit Et₂O und nachfolgender Kristallisation ***2*** isolieren ließ. Die Ausbeute hierfür betrug 57 %. Durch Röntgendiffraktometrie konnte die Kristallstruktur von ***2*** ermittelt werden, dargestellt in **Abb. 2a****.**

Alle Ergebnisse einer ¹H, ¹³C und ²⁹Si NMR-spektroskopischen Untersuchung:
**²⁹Si NMR (99,4 MHz, CH₂Cl₂, 298 K):**
   δ = 12,3
**¹H NMR (500,2 MHz, CH₂Cl₂, 298 K):**
   δ = 7,41 (t ³J(H,H) = 7,2 Hz (2H)), 7,47 (d ³J(H,H) = 7,2 Hz (1H)), 7,56 (d ³J(H,H) = 7,2 Hz (2H)).
**¹³C NMR (125,0 MHz, CH₂Cl₂, 298 K):**
   δ = 129,0; 130,1; 131,8; 136,0.

### Beispiel 3: Herstellung von Trichlorsilyltrichlorgerman (3).

Die Synthese erfolgte gemäß **Gleichung 3** aus GeCl₄ und Si₂Cl₆ (1:1) unter Zugabe von 0,1 eq. [*n*Bu₄N]Cl als katalytische Menge.

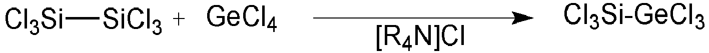

**Gleichung 3**: Umsetzung von GeCl₄ und Si₂Cl₆ (1:1) unter Zugabe von katalytischer Menge (0,1eq.) des Katalysators mit R = Bu: [*n*Bu₄N]Cl. Zu einer klaren, farblosen Lösung enthaltend 100 mg oder 0,4 mmol [*n*Bu₄N]Cl in einem 30:70 Gemisch aus GeCl₄ und CH₂Cl₂ wurde bei Raumtemperatur 1 g oder 3,7 mmol Si₂Cl₆ gegeben und das so erhaltene Reaktionsgemisch bei Raumtemperatur während 12 h gerührt. Aus der Reaktionslösung wurde das Produkt ***3*** mit anderen flüchtigen Bestandteilen in eine stickstoffgekühlte Vorlage kondensiert. Eine anschließende Destillation bei 140 °C bei 1013 hPa ergab die Isolation von reinem ***3*** als klare farblose Flüssigkeit in einer Ausbeute von 22%.

Das ²⁹Si NMR Spektrum von ***3*** ist in **Abb. 3a** dargestellt.

Alle Ergebnisse einer ²⁹Si NMR-spektroskopischen Untersuchung:
**²⁹Si NMR (99,4 MHz; CH₂Cl₂; 298 K):**
δ = -6,3.

### Beispiel 4: Herstellung von Trichlorogermanid (4).

Es wurde wie im Beispiel 3 vorgegangen, jedoch mit dem Unterschied, dass [*n*Bu₄N]Cl in stöchiometrischer Menge gleichbedeutend 1 eq. eingesetzt wurde. Die Umsetzungsreaktion erfolgte in einer Redox-Reaktion gemäß **Gleichung 4.** 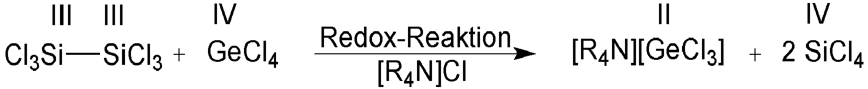 **Gleichung 4:** Umsetzung von GeCl₄ und Si₂Cl₆ (1:1) unter Zugabe von stöchiometrischer Menge, nämlich 1 eq., an [*n*R₄N]Cl mit R = Bu.

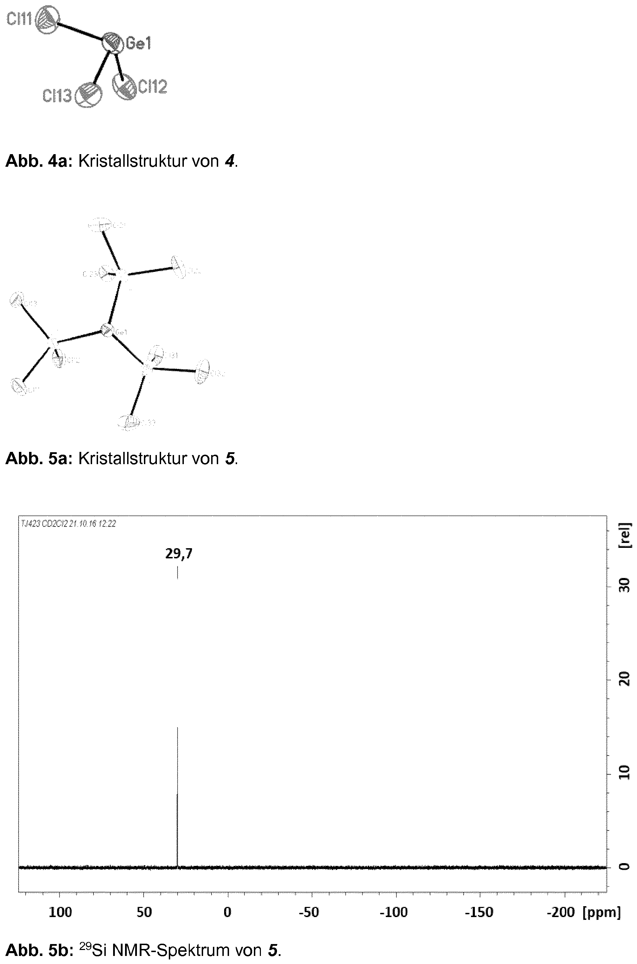

Zu einer klaren farblosen Lösung aus 300 mg oder 1,4 mmol GeCl₄ und 390 mg oder 1,4 mmol [*n*Bu₄N]Cl in CH₂Cl₂ wurden bei Raumtemperatur 375 mg oder 1,4 mmol Si₂Cl₆ gegeben und das so erhaltene Reaktionsgemisch während 12 h gerührt. Aus der Reaktionslösung ließ sich nach langsamem Entfernen des Lösemittels Trichlorogermanid ***4*** als gelblicher, kristalliner Feststoff isolieren. Durch Röntgendiffraktometrie konnte die Struktur von ***4*** ermittelt werden, gezeigt in **Abb. 4a**. Das Kation [*n*Bu₄N]⁺ ist hier aus Gründen der Übersichtlichkeit nicht abgebildet.

### Beispiel 5: Herstellung von Tris-Trichlorsilylgermanid (5).

Die Synthese erfolgte gemäß **Gleichung** 5 aus GeCl₄ und Si₂Cl₆ in einem molaren Verhältnis von 1:4 unter Zugabe einer stöchiometrischen Menge, in diesem Fall 1 eq., von [Ph₄P]Cl. 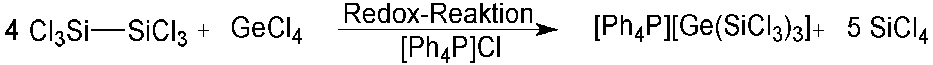 **Gleichung 5:** Umsetzung von GeCl₄ und Si₂Cl₆ (1:4) unter Zugabe von stöchiometrischer Menge (1 eq.) an [Ph₄P]Cl.

Zu einer klaren, farblosen Lösung enthaltend 93 mg oder 0,4 mmol GeCl₄ und 163 mg oder 0,4 mmol [Ph₄P]Cl in CH₂Cl₂ als Lösemittel wurden bei Raumtemperatur 478 mg oder 1,7 mmol Si₂Cl₆ gegeben und das so erhaltene Reaktionsgemisch während 12 h gerührt. Aus diesem Gemisch ließ sich nach langsamem Entfernen des Lösemittels ***5*** als gelblicher, kristalliner Feststoff in 99 % Ausbeute isolieren. Durch Röntgendiffraktometrie konnte die Kristallstruktur von ***5*** ermittelt werden, gezeigt in **Abb. 5a****.** Das Kation [Ph₄P]⁺ ist hier aus Gründen der Übersichtlichkeit nicht abgebildet.

Bei dieser Reaktion kommt es zu einer Reduktion des GeCl₄. Zudem erfolgt dreifach Silylierung am Germaniumatom.

Das ²⁹Si NMR Spektrum von ***5*** ist in **Abb. 5b** dargestellt.

Alle Ergebnisse einer ¹H und ²⁹Si NMR-spektroskopischen Untersuchung:
**²⁹Si NMR (99,4 MHz; CD₂Cl₂; 298 K):**
   δ = 29,7.
**¹H NMR (500,2 MHz; CD₂Cl₂; 298 K):**
   δ = 7,54 (qd ³J(H,H) = 4,5 Hz; ²J(H,H) = 1,3 Hz; (2H)); 7,68 (td ³J(H,H) =7,4 Hz; ³J(H,H) = 3,4 Hz (2H)); 7,84 (tt ³J(H,H) = 7Hz; ²J(H,H) = 1,0 Hz (1H)).

### Beispiel 6: Herstellung von Tris(trichlorsilyl)germanid an GaCl₃ (6) Addukt.

Die Synthese von ***6*** erfolgte gemäß **Gleichung 6** aus Tris-Trichlorsilylgermanid (***5***) und GaCl₃.

[Ph₄P][Ge(SiCl₃)₃] + GaCl₃ → [Ph₄P][Ge(SiCl₃)₃*GaCl₃]

**Gleichung 6:** Umsetzung von ***5*** mit GaCl₃.

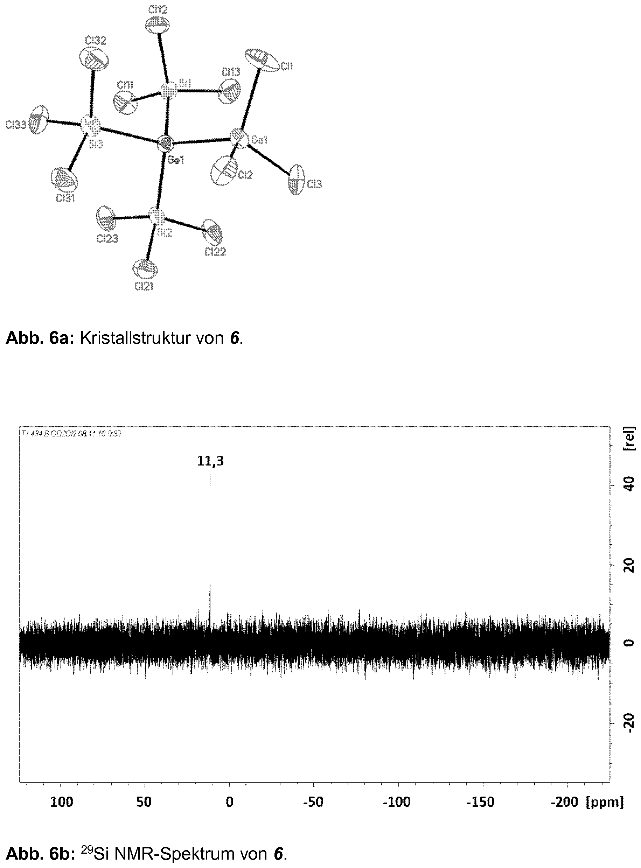

50 mg oder 0,1 mmol ***5*** und 10 mg oder 0,1 mmol GaCl₃ wurden als Feststoffe bei Raumtemperatur vermischt und anschließend vollständig in CH₂Cl₂ gelöst. Das klare, gelbe Reaktionsgemisch wurde während 12 h bei Raumtemperatur gerührt. Aus der klaren, gelben Reaktionslösung ließ sich nach langsamem Entfernen des Lösemittels ***6*** als gelber kristalliner Feststoff in einer Ausbeute von 82 % erhalten. Durch Röntgendiffraktometrie konnte die Kristallstruktur von ***6*** ermittelt werden, gezeigt in **Abb. 6a****.** Das Kation [Ph₄P]⁺ ist hier aus Gründen der Übersichtlichkeit nicht abgebildet.

### Das ²⁹Si NMR Spektrum von 6 ist in Abb. 6b dargestellt.

Alle Ergebnisse einer ¹H und ²⁹Si NMR-spektroskopischen Untersuchung:
**²⁹Si NMR (99,4 MHz; CD₂Cl₂; 298 K):**
   δ = 11,3.
**¹H NMR (500,2 MHz; CD₂Cl₂; 298 K):**
   δ = 7,54 (qd ³J(H,H) = 4,5 Hz; ²J(H,H) = 1,3 Hz; (2H)); 7,68 (td ³J(H,H) =7,4 Hz; ³J(H,H) = 3,4 Hz (2H)); 7,84 (tt ³J(H,H) = 7Hz; ²J(H,H) = 1,0 Hz (1H)).

### Beispiel 7: Herstellung von Tris(trichlorsilyl)germanid an BBr₃ (7) Addukt.

Die Synthese von ***7*** erfolgte gemäß **Gleichung 7** aus Tris-Trichlorsilylgermanid (***5***) und BBr₃. 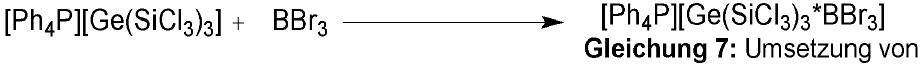 ***5*** mit BBr₃.

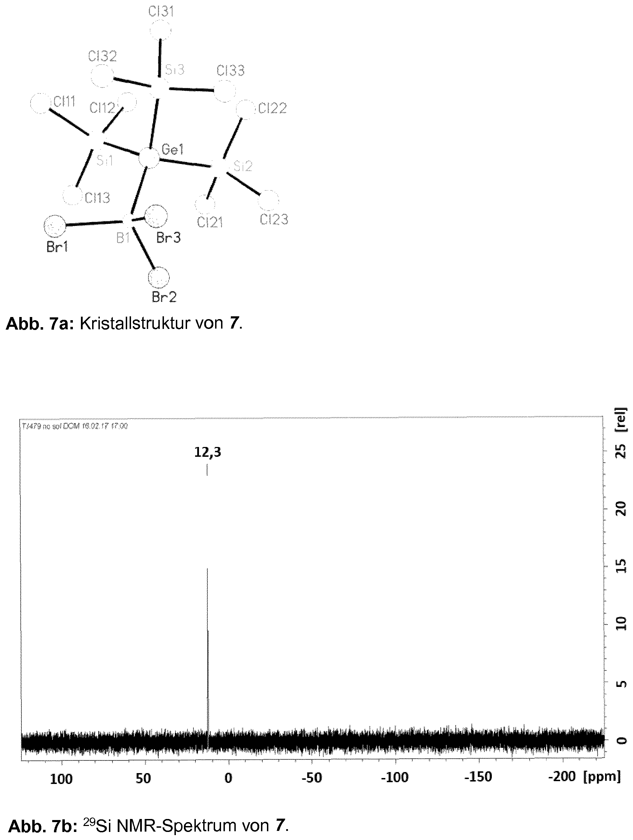

Zu einer klaren gelben Lösung von ***5*** in CH₂Cl₂ wurde bei Raumtemperatur BBr₃ gegeben. Nach 4 Tagen waren farblose Kristalle aus der so erhaltenen gelben Reaktionslösung ausgefallen. Durch Röntgendiffraktometrie konnte die Kristallstruktur von ***7*** ermittelt werden, gezeigt in **Abb. 7a****.** Das Kation [Ph₄P]⁺ ist hier aus Gründen der Übersichtlichkeit nicht abgebildet.

Das ²⁹Si NMR Spektrum von ***7*** ist in **Abb. 7b** dargestellt.

Alle Ergebnisse einer ¹¹B und ²⁹Si NMR-spektroskopischen Untersuchung
**²⁹Si NMR (99,4 MHz; CD₂Cl₂; 298 K):**
δ = 12,3.
**¹¹B NMR (160,5 MHz; CD₂Cl₂; 298 K):**
δ = -17,5.

## Patentansprüche

1. Verfahren zur Herstellung von Trichlorogermanid gemäß der allgemeinen Formel I,
(I) [R₄N]/[R₄P]Cl[GeCl₃],
mit R = Me, Et, *i*Pr, *n*Bu, Ph, indem man
GeCl₄ mit Hexachlordisilan in einem Lösemittel löst und
in Gegenwart von [R₄N]/[R₄P]Cl in stöchiometrischer Menge bei einer Temperatur von 5 bis 40 °C umsetzt.

2. Verfahren zur Herstellung von Tris-Trichlorsilylgermanid gemäß der allgemeinen Formel II,
(II) [R₄N]/[R₄P][Ge(SiCl₃)₃],
indem man
Hexachlordisilan mit GeCl₄ im Verhältnis von 4:1 in einem Lösemittel löst und in Gegenwart von [R₄N]/[R₄P]Cl in stöchiometrischer Menge bei einer Temperatur von 5 bis 40 °C umsetzt.

3. Verfahren zur Herstellung von Tris(trichlorsilyl)germanid an GaCl₃ Addukt der Formel III
(III) [Ph₄P][Ge(SiCl₃)₃*GaCl₃],
indem man
- Tris-Trichlorsilylgermanid gemäß der allgemeinen Formel II,
(II) [R₄N]/[R₄P][Ge(SiCl₃)₃],
dadurch erhalten, dass man
Hexachlordisilan mit GeCl₄ im Verhältnis von 4:1 in einem Lösemittel löst und in Gegenwart von [R₄N]/[R₄P]Cl in stöchiometrischer Menge bei einer Temperatur von 5 bis 40 °C umsetzt,
mit GaCl₃ vermischt und das erhaltene Gemisch
- in Dichlormethan unter Rühren löst, und anschließend bei einer Temperatur von 5 bis 40 °C während 1 bis 24 Stunden das Addukt III erhalten wird.

4. Verfahren zur Herstellung von Tris(trichlorsilyl)germanid an GaCl₃ Addukt der Formel IV
(IV) [Ph₄P][Ge(SiCl₃)₃*BBr₃],
indem man
- Tris-Trichlorsilylgermanid gemäß der allgemeinen Formel II,
(II) [R₄N]/[R₄P][Ge(SiCl₃)₃],
dadurch erhalten, dass man
Hexachlordisilan mit GeCl₄ im Verhältnis von 4:1 in einem Lösemittel löst und in Gegenwart von [R₄N]/[R₄P]Cl in stöchiometrischer Menge bei einer Temperatur von 5 bis 40 °C umsetzt,
mit BBr₃ vermischt und das erhaltene Gemisch
- in Dichlormethan unter Rühren löst, und anschließend bei einer Temperatur von 5 bis 40 °C während 1 bis 120 Stunden das Addukt IV erhalten wird.

## Claims

1. Method for preparing a trichlorogermanide of the general formula I
(I) [R₄N]/[R₄P]Cl[GeCl₃],
where R = Me, Et, *i*Pr, *n*Bu, Ph,
by dissolving GeCl₄ with hexachlorodisilane in a solvent and reacting in the presence of a stoichiometric amount of [R₄N]/[R₄P]Cl at a temperature of 5 to 40°C.

2. Method for preparing a tris(trichlorosilyl)germanide of the general formula II
(II) [R₄N]/[R₄P][Ge(SiCl₃)₃]
by dissolving hexachlorodisilane with GeCl₄ at a ratio of 4:1 in a solvent and reacting in the presence of a stoichiometric amount of [R₄N]/[R₄P]Cl at a temperature of 5 to 40°C.

3. Method for preparing the tris(trichlorosilyl)germanide adduct of GaCl₃ of the formula III
(III) [Ph₄P] [Ge (SiCl₃)₃*GaCl₃],
in which
- tris(trichlorosilyl)germanide of the general formula II,
(II) [R₄N]/[R₄P][Ge (SiCl₃)₃]
is obtained by dissolving hexachlorodisilane with GeCl₄ at a ratio of 4:1 in a solvent and reacting in the presence of a stoichiometric amount of [R₄N]/[R₄P]Cl at a temperature of 5 to 40°C,
mixing with GaCl₃ and dissolving the resulting mixture
- in dichloromethane with stirring, and subsequently obtaining the adduct III at a temperature of 5 to 40°C over a period of 1 to 24 hours.

4. Method for preparing the tris(trichlorosilyl)germanide adduct of GaCl₃ of the formula IV
(IV) [Ph₄P] [Ge (SiCl₃)₃*BBr₃],
in which
- tris(trichlorosilyl)germanide of the general formula II,
(II) [R₄N]/[R₄P][Ge (SiCl₃)₃]
is obtained by dissolving hexachlorodisilane with
GeCl₄ at a ratio of 4:1 in a solvent and reacting in the presence of a stoichiometric amount of [R₄N]/[R₄P]Cl at a temperature of 5 to 40°C,
mixing with BBr₃ and dissolving the resulting mixture
- in dichloromethane with stirring, and subsequently obtaining the adduct IV at a temperature of 5 to 40°C over a period of 1 to 120 hours.

## Revendications

1. Procédé pour la préparation de trichlorogermanure selon la formule générale I,
(I) [R₄N]/[R₄P]Cl[GeCl₃],
avec R = Me, Et, *i*Pr, *n*Bu, Ph,
par le fait que l'on
dissout du GeCl₄ avec de l'hexachlorodisilane dans un solvant et on transforme en présence de [R₄N]/[R₄P]Cl en quantité stœchiométrique à une température de 5 à 40 °C.

2. Procédé pour la préparation de tris-trichlorosilylgermanure selon la formule générale II,
(II) [R₄N]/[R₄P][Ge(SiCl₃)₃],
par le fait que l'on
dissout de l'hexachlorodisilane avec du GeCl₄ en un rapport 4 : 1 dans un solvant et on transforme en présence de [R₄N]/[R₄P]Cl en quantité stœchiométrique à une température de 5 à 40 °C.

3. Procédé pour la préparation d'adduit de tris(trichlorosilyl)germanure avec GaCl₃ de formule III
(III) [Ph₄P] [Ge(SiCl₃)₃*GaCl₃],
par le fait que
- on mélange un tris-trichlorosilylgermanure de formule générale II,
(II) [R₄N]/[R₄P][Ge(SiCl₃)₃]
obtenu en
dissolvant de l'hexachlorodisilane avec du GeCl₄ en un rapport de 4 : 1 dans un solvant et en transformant en présence de [R₄N]/[R₄P]Cl en quantité stœchiométrique à une température de 5 à 40 °C,
avec du GaCl₃ et
- on dissout le mélange obtenu dans du dichlorométhane sous agitation, et ensuite l'adduit III est obtenu à une température de 5 à 40 °C pendant 1 à 24 heures.

4. Procédé pour la préparation d'adduit de tris(trichlorosilyl)germanure avec GaCl₃ de formule IV
(IV) [Ph₄P] [Ge(SiCl₃)₃*BBr₃],
par le fait que
- on mélange un tris-trichlorosilylgermanure de formule générale II,
(II) [R₄N]/[R₄P][Ge (SiCl₃)₃]
obtenu en
dissolvant de l'hexachlorodisilane avec du GeCl₄ en un rapport de 4 : 1 dans un solvant et en transformant en présence de [R₄N]/[R₄P]Cl en quantité stœchiométrique à une température de 5 à 40 °C,
avec du BBr₃ et
- on dissout le mélange obtenu dans du dichlorométhane sous agitation, et ensuite l'adduit IV est obtenu à une température de 5 à 40 °C pendant 1 à 120 heures.
